# EUROPÄISCHE PATENTSCHRIFT

(11) **EP 0 490 815 B1**
(45) Veröffentlichungstag und Bekanntmachung des Hinweises auf die Patenterteilung: **21.09.1994**
(21) Anmeldenummer: 91810939.8
(22) Anmeldetag: 03.12.1991
(51) Int. Cl.: C07D 249/20

(54) **Verfahren zur Herstellung von symmetrischen 2,2'-Methylen-bis-benztriazolylphenolen**
Process for the preparation of symmetric 2,2'-methylene-bis-benzotriazolyl-phenols
Procédé pour la préparation de 2,2'-méthylène-bis-benzotriazolyl-phénols symétriques

(30) Priorität: 11.12.1990 CH 3903/90
(43) Veröffentlichungstag der Anmeldung: 17.06.1992
(73) Patentinhaber: CIBA-GEIGY AG, 4002 Basel (CH)
(72) Erfinder: Luisoli, Reto, CH-4434 Hölstein (CH); Stegmann, Werner, Dr., CH-4410 Liestal (CH)

(56) Entgegenhaltungen:
- EP-A- 0 180 993

## Beschreibung

Die vorliegende Erfindung betrifft ein neues Verfahren zur Herstellung von 2,2'-Methylen-bis-benztriazolylphenolen.

Ein Verfahren zur Herstellung solcher Verbindungen ist z.B. aus der EP-A-180,993 bekannt. Danach wird in einer ersten Reaktionsstufe ein Benztriazolylphenol mit einem Amin und Formaldehyd in einem organischen Lösungsmittel zur entsprechenden Mannichbase umgesetzt. Nach Isolierung der Mannichbase erfolgt in einer zweiten Reaktionsstufe ihre Umsetzung mit weiterem Benztriazolylphenol in einem anderen organischen Lösungsmittel zu dem gewünschten 2,2'-Methylen-bis-benztriazolylphenol.

Es wurde nun gefunden, dass symmetrische 2,2'-Methylen-bis-benztriazolylphenole auf einfachere und schnellere Weise zuganglich sind als bisher, wenn man die Umsetzung von Benztriazolylphenol, Amin und Formaldehyd in Abwesenheit organischer Lösungsmittel durchführt.

Gegenstand der vorliegenden Erfindung ist somit ein Verfahren zur Herstellung von symmetrischen 2,2'-Methylen-bis-benztriazolylphenolen der Formel
(1) 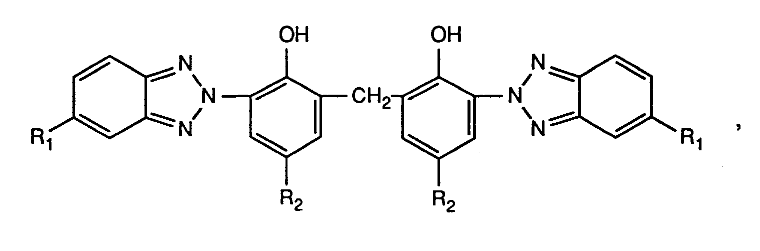 worin
   R₁ Wasserstoff, Alkyl oder Alkoxy mit 1 bis 12 Kohlenstoffatomen, Phenyl, Phenylalkyl mit 1 bis 4 Kohlenstoffatomen im Alkylteil oder Halogen und
   R₂ Alkyl mit 1 bis 12 Kohlenstoffatomen, mit CO₂H-Gruppen substituiertes Alkyl mit 1 bis 12 Kohlenstoffatomen, Phenyl, Phenylalkyl mit 1 bis 4 Kohlenstoffatomen im Alkylteil oder Cycloalkyl mit 5 bis 8 Kohlenstoffatomen ist, wobei man eine Verbindung der Formel
(2) 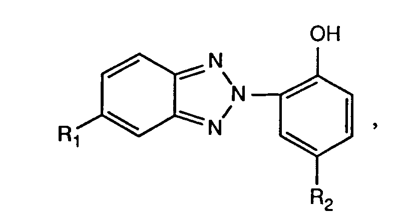 worin R₁ und R₂ die angegebenen Bedeutungen haben, mit einer Verbindung der Formel
(3) 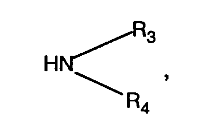 worin R₃ und R₄ unabhangig voneinander Alkyl mit 1 bis 4 Kohlenstoffatomen sind, und der Verbindung der Formel
(4) CH₂O (Formaldehyd) oder einer polymeren Form davon zur entsprechenden Mannichbase umsetzt und letztere mit einer Base behandelt, dadurch gekennzeichnet, dass beide Reaktionen als Eintopfverfahren in Abwesenheit eines Losungsmittels durchgeführt werden.

Die Verbindungen der Formel (1) sind bekannt, beispielsweise aus der genannten EP-A-180,993. Die Ausgangsverbindungen der Formel (2) sind ebenfalls bekannt und z.B. in US-A-4,642,350 und EP-A-31,302 mit Herstellungsmethoden beschrieben.

Die 2,2'-Methylen-bis-benztriazolylphenole der Formel (1) können z.B. als Lichtschutzmittel in organischen Polymeren verwendet werden.

In den erfindungsgemäss hergestellten Verbindungen der Formel (1) ist der Substituent R₁ neben Wasserstoff Alkyl und 1 bis 12 Kohlenstoffatomen, beispielsweise Methyl, Äthyl, Propyl, Butyl, Hexyl, Octyl, Nonyl, Decyl und Dodecyl sowie entsprechende verzweigte Isomere. Beispiele geeigneter Alkoxyreste lassen sich aus dieser Aufzahlung ableiten. Des weiteren ist R₁ Phenyl oder Phenylalkyl, wobei der Alkylteil 1 bis 4 Kohlenstoffatome enthalt. Beispiele hierfür sind Phenyläthyl und -propyl α-Methylbenzyl, α,α-Dimethylbenzyl und, vorzugsweise, Benzyl. R₁ kann auch Halogen bedeuten wie Chlor oder Brom. Bevorzugt ist R₁ Wasserstoff, Alkyl mit 1 bis 4 Kohlenstoffatomen oder Chlor.

Für R₂ kommen die unter R₁ genannten Alkylreste in Frage. Diese können mit CO₂H-Gruppen substituiert sein, um Gruppierungen wie etwa -CH₂CH₂CO₂H zu bilden. Neben Phenyl und Phenylalkyl mit 1 bis 4 Kohlenstoffatomen im Alkylteil (Beispiele wie für R₁) kann R₂ auch Cycloalkyl mit 5 bis 8 Kohlenstoffatomen bedeuten, z.B. Cyclopentyl, Cyclohexyl und Cyclooctyl.

R₃ und R₄ in den Verbindungen der Formel (3) sind unabhängig voneinander Alkyl mit 1 bis 4 Kohlenstoffatomen wie Methyl, Propyl und Butyl sowie entsprechende verzweigte Isomere.

Vorzugsweise werden solche Verbindungen der Formel (2) eingesetzt, worin R₁ Wasserstoff, Alkyl mit 1 bis 4 Kohlenstoffatomen oder Halogen, insbesondere Chlor, und R₂ Alkyl mit 1 bis 12 Kohlenstoffatomen ist, und insbesondere R₁ Wasserstoff und R₂ Alkyl mit 6 bis 12 Kohlenstoffatomen ist. Ein besonders bevorzugter Alkylrest R₂ ist t-Octyl.

R₃ und R₄ in den Verbindungen der Formel (3) haben vorzugsweise dieselbe Bedeutung und sind insbesondere Methyl oder Butyl.

Neben Formaldehyd können als Verbindung der Formel (4) auch Polymere des Formaldehyds wie Paraformaldehyd [(CH₂O)ₙ, n= 12-30] oder Trioxan verwendet werden.

Das erfindungsgemässe Verfahren kann beispielsweise so ausgeführt werden, dass man zweckmässigerweise unter Unterdruck, z.B. 1 bis 50 mbar, ein Benztriazolylphenol und Formaldehyd vorlegt und diese Mischung in eine Schmelze überführt. In diese Schmelze wird das Dialkylamin eingeleitet, wonach man bei etwas höherer Temperatur, z.B. 120 bis 150°C, ausreagieren lässt. Der Druck im Reäktionsgefäss kann während dieser Phase auf etwa 1700 bis 1900 mbar steigen. Ueblicherweise kann die Reaktion bereits nach 2 bis 4 Stunden abgebrochen werden. Man lässt die Schmelze, die nun die entsprechende Mannichbase und unumgesetztes Benztriazolylbenzol enthält, abkühlen und entfernt das gebildete Reaktionswasser sowie überschüssiges Amin durch Anlegen von Vakuum. Die Mannichbase wird nicht isoliert; sie wird in demselben Reaktionsgefäss weiter umgesetzt. Zur Aminabspaltung wird der Reaktionsmischung eine Base, z.B. ein Alkalimetallalkoholat wie Natriummethanolat oder -äthanolat, oder ein Alkalimetallhydroxid wie Natrium- oder Kaliumhydroxid` hinzugefügt und unter Unterdruck, z.B. 150 bis 200 mbar auf etwa 180 bis 220°C aufgeheizt. In der Regel ist die Aminabspaltung schon nach 2 bis 4 Stunden beendet. Zur Neutralisation der Base wird die Schmelze in einem höher siedenden organischen Lösungsmittel wie Xylol aufgenommen und mit einer Carbonsäure, vorzugsweise Ameisen- oder Essigsäure, versetzt Nach Klärfiltration erfolgt die Aufarbeitung des Reaktionsprodukts durch übliche Methoden wie Kristallisation, Destillation oder Extraktion. Die Schmelzkristallisation als Aufarbeitungs- bzw. Reinigungsverfahren kann sich direkt an die Neutralisation mit Carbonsäure anschliessen. In diesem Fall verzichtet man auf die Zugabe des organischen Lösungsmittels. Unumgesetztes Benztriazolylphenol kann durch Destillation, z.B. mittels Dünnschichtverdampfer, aus dem höher siedenden organischen Lösungsmittel zurückgewonnen werden. Dadurch erhöht sich die auf verbrauchtes Benztriazolylphenol bezogene Ausbeute in der Regel um 4 bis 10 %. Das Lösungsmittel kann recycliert werden.

Charakteristisch für das erfindungsgemässe Verfahren ist seine Ausführung als Eintopfverfahren in Abwesenheit organischer Lösungsmittel.

Diese Reaktionsführung bringt neben einer Ausbeutesteigerung an Reinprodukt gegenüber dem in EP-A-180,993 als Stand der Technik beschriebenen Verfahren weitere bedeutende Vorteile. Verglichen mit diesem Stand der Technik verkürzt sich nämlich erfindungsgemäss die reine Reaktionszeit von insgesamt 34 Stunden (24 Stunden für 1.Stufe, 10 Stunden für 2.Stufe) auf 4 bis 8 Stunden. Dies bedeutet, dass das erfindungsgemässe Verfahren in nur dem sechsten bis achten Teil der Zeit, die in EP-A-180,993 allein schon für die erste Reaktionsstufe benötigt wird, zu höheren Ausbeuten an Produkt führt, ohne dass es zu Einbussen bezüglich Reinheit des Produktes kommt. Die Raum-Zeit-Ausbeute wird erfindungsgemäss um mindestens den Faktor 5 verbessert.

Erfindungsgemäss ist auch die Isolierung der Zwischenstufe (Mannichbase der Formel 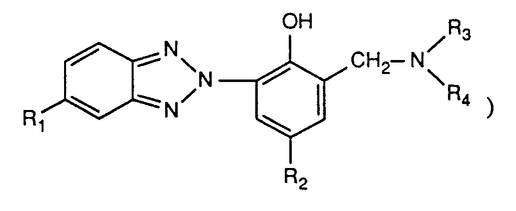
oder gar Reinigung dieser Zwischenstufe nicht notwendig.

Gemäss EP-A-180,993 werden für die erste und zweite Reaktionsstufe jeweils verschiedene organische Lösungsmittel verwendet. Die damit verbundenen Schwierigkeiten, d.h. Reinigung der Lösungsmittel oder Entsorgung, treten beim Verfahren der vorliegenden Erfindung nicht auf.

Diese Vorteile lassen klar erkennen, dass es sich bei dem Verfahren gemäss vorliegender Erfindung um eine einfache und sehr wirtschaftliche Methode zur Herstellung von 2,2'-Methylen-bis-benztriazolylphenolen handelt.

Vorzugsweise wird das erfindungsgemässe Verfahren bei einer Temperatur von 60 bis 300, insbesondere 130 bis 220°C durchgeführt. Dabei erfolgt die erste Stufe bis zur Darstellung der Mannichbase bei 60 bis 150, vorzugsweise 120 bis 150°C, die zweite Stufe bis zur Bildung des Endprodukts bei 180 bis 300, vorzugsweise 180 bis 220°C. In der zweiten Stufe ist die Anwesenheit einer Base, z.B. eines Alkalimetallalkoholats oder -hydroxids, erforderlich. Die Molverhältnisse der Verbindungen der Formel (2), (3) und (4) betragen bevorzugt 2:1:1 bis 2:3:3, insbesondere bis 2:2,5:1,5.

Besonders geeignet ist das erfindungsgemässe Verfahren zur Herstellung von 2,2'-Methylen-bis-[4-(1,1,3,3-tetramethylbutyl)-6-benztriazolylphenol] durch Umsetzung der Verbindung der Formel (2), worin R₁ Wasserstoff und R₂ t-Octyl ist, mit einer Verbindung der Formel (3), worin R₃ und R₄ gleichzeitig Methyl oder Butyl sind, und Formaldehyd, wobei diese Verbindungen im Molverhältnis von 2:2:1 bis 2:2,2:1,1 eingesetzt werden, und die Reaktionstemperatur 130 bis 220°C beträgt.

Die folgenden Beispiele erläutern die Erfindung weiter. Prozentangaben beziehen sich auf das Gewicht.

Beispiel 1: In einem 0,75 l Doppelmantelkolben, geprüft auf 1,5 bar Ueberdruck, ausgestattet mit Musterschleuse für Gaseinleitung, absteigendem Kühler mit Auffanggefäss und Vakuumleitung mii Kühlfalle zur Vakuumpumpe, werden 323,2 g (1,0 mol) 4-(1,1 ,3,3-Tetramethyl)butyl-6-benztriazol-2-ylphenol und 16,5 g (0,55 mol) Paraformaldehyd vorgelegt. Das angesetzte Gemisch wird nach Evakuieren auf 20 mbar und Schliessen des Kolbens bei einer Manteltemperatur von 120°C geschmolzen. Dabei steigt der Druck bis etwa 270 mbar an. Bei 100 bis 105°C werden in die gut rührbare Schmelze 24,8 g (0,55 mol) Dimethylamingas während 30 Minuten eingeleitet. Es stellt sich ein Enddruck von 900 bis 1000 mbar ein. Das Reaktionsgemisch wird auf 135°C aufgeheizt und 2 bis 4 Stunden bei dieser Temperatur gerührt. Der Druck steigt dabei bis 1800 mbar. Nach dem Abkühlen auf 90°C wird das Reaktionswasser zusammen mit nichtumgesetztem Amin durch Anlegen von Vakuum und Aufheizen auf 130°C entfernt. Nach Entlasten mit Stickstoff werden 2,2 g (0,04 mol) Natriummethylat als Katalysator zur Reaktionsmasse gegeben und diese nach Anlegen eines Vakuums von 200 mbar rasch auf 200°C aufgeheizt. Ab etwa 145 bis 155°C ist die Abspaltung von Dimethylamin zu beobachten. Nach 2- bis 4-stündigem Ausrühren bei 200°C und etwa 200 mbar kommt die Aminabspaltung zum Stillstand und die Reaktion ist beendet. Nach dem Lösen der Schmelze in 200 g Xylolgemisch wird der alkalische Katalysator mit 3,2 ml Ameisensäure (85 %) neutralisiert, die Lösung bei 130°C klärfiltriert und mit 75 g Xylolgemisch nachgewaschen. Die Kristallisation erfolgt durch Abkühlen auf etwa 0°C. Die dickflüssige Suspension wird abgenutscht, mit 100 g Xylolgemisch gewaschen und das Produkt im Vakuumofen bei 120°C getrocknet. Man erhält 294 g Produkt in Form eines gelblichen Pulvers (89,3 % derTheorie bezogen auf eingesetztes Benztriazolylphenol, Fp: 197,6°C, Transmission (5 % in Chloroform) bei 450 nm: 96,4 %, bei 500 nm: 97,7 %).

Die Waschlauge kann vollständig eingedampft und der verbleibende Rückstand auf einem Dünnschichtverdampfer so andestilliert werden, dass das nicht umgesetzte Benztriazolylphenol quantitativ zurückgewonnen werden kann. So können 4 bis 10 % des eingesetzten Benztriazolylphenols wiedergewonnen und im nächsten Versuch eingesetzt werden. Damit beträgt die Ausbeute bezogen auf verbrauchtes Benztriazolylphenol 90 bis 92 % der Theorie.

Beispiel 2: In einem 0,75 l Sulfierkolben mit Ankerrührer, Rückflusskühler, Stickstoffbegasung und Oelbad werden 323,2 g (1,0 mol) 4-(1,1,3,3-Tetramethyl)butyl-6-benztriazol-2-yl-phenol und 16,5 g (0,55 mol) Paraformaldehyd zusammen mit 142,2 g (1,1 mol) Dibutylamin vorgelegt. Die Suspension wird auf etwa 100°C aufgeheizt und bei 100 bis 102°C 6 Stunden gerührt. Anschliessend werden der Ueberschuss an Dibutylamin und das entstandene Reaktionswasser durch Destillation bei 5 mbar und 120°C entfernt. Die danach folgende Arbeitsweise entspricht der in Beispiel 1 angegebenen, wobei bei einem Druck von 5 mbar gearbeitet wird und die Reaktionsdauer nur 1 bis 2 Stunden beträgt. Man erhält 277 bis 290 g Produkt in Form eines gelblichen Pulvers (84 bis 88 % der Theorie bezogen auf eingesetztes Benztriazolylphenol, Fp: 197°C, Transmission (5 % in Chloroform) bei 450 nm: 97,0 %, bei 500 nm: 98,8 %).

Die Mutterlauge kann wie in Beispiel 1 aufgearbeitet werden. Die Ausbeute steigt dann auf 88 bis 92 % der Theorie.

Beispiel 3: Im 0,75 l Doppelmanteldruckreaktor gemäss Beispiel 1, werden 323,2 g (1,0 mol) 4-(1,1,3,3-Tetramethyl)butyl-6-benztriazol-2-yl-phenol mit 15,6 g (0,52 mol) Paraformaldehyd und 134,4 g (1,04 mol) Dibutylamin vorgelegt, der Reaktor auf 100 mbar evakuiert und verschlossen. Die Suspension wird auf 135°C aufgeheizt und während einer Stunde bei dieser Temperatur gerührt. Der Druck steigt dabei auf etwa 1,7 bar. Die weiteren Schritte werden analog Beispiel 1 durchgeführt, wobei die Katalysatormenge auf 0,01 Mol entsprechend 0,54 g oder 1 Mol% reduziert wird.

Man erhält 277 bis 290 g Produkt in Form eines gelblichen Pulvers (84 bis 88 % der Theorie bezogen auf das eingesetzte Benztriazolylphenol, oder 88 bis 92 % bez. auf verbrauchtes Benztriazolylphenol, Fp: 197,5°C, Transmission (5 % in Chloroform) bei 450 nm: 97,9 %, bei 500 nm: 99,4 %).

## Patentansprüche

1. Verfahren zur Herstellung von symmetrischen 2,2'-Methylen-bis-benztriazolylphenolen der Formel
(1) 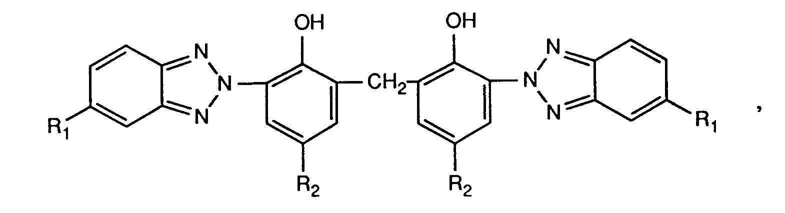 worin
R₁ Wasserstoff, Alkyl oder Alkoxy mit 1 bis 12 Kohlenstoffatomen, Phenyl, Phenylalkyl mit 1 bis 4 Kohlenstoffatomen im Alkylteil oder Halogen und
R₂ Alkyl mit 1 bis 12 Kohlenstoffatomen, mit CO₂H-Gruppen substituiertes Alkyl mit 1 bis 12 Kohlenstoffatomen, Phenyl, Phenylalkyl mit 1 bis 4 Kohlenstoffatomen im Alkylteil oder Cycloalkyl mit 5 bis 8 Kohlenstoffatomen ist, wobei man eine Verbindung der Formel
(2) 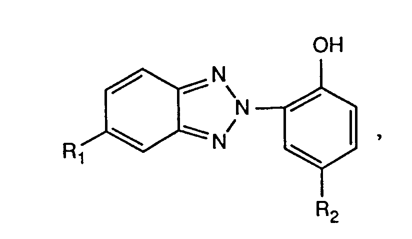 worin R₁ und R₂ die angegebenen Bedeutungen haben, mit einer Verbindung der Formel
(3) 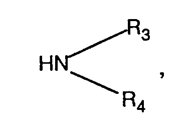 worin R₃ und R₄ unabhängig voneinander Alkyl mit 1 bis 4 Kohlenstoffatomen sind, und der Verbindung der Formel
(4) CH₂O (Formaldehyd) oder einer polymeren Form davon zur entsprechenden Mannichbase umsetzt und letztere mit einer Base behandelt, dadurch gekennzeichnet, dass beide Reaktionen als Eintopfverfahren in Abwesenheit eines Lösungsmittels durchgeführt werden.

2. Verfahren nach Anspruch 1, dadurch gekennzeichnet, dass R₁ Wasserstoff, Alkyl mit 1 bis 4 Kohlenstoffatomen oder Halogen und R₂ Alkyl mit 1 bis 12 Kohlenstoffatomen ist.

3. Verfahren nach Anspruch 2, dadurch gekennzeichnet, dass R₁ Wasserstoff und R₂ Alkyl mit 6 bis 12 Kohlenstoffatomen ist.

4. Verfahren nach Anspruch 3, dadurch gekennzeichnet, dass R₂ t-Octyl ist.

5. Verfahren nach Anspruch 1, dadurch gekennzeichnet, dass R₃ und R₄ Methyl oder Butyl sind.

6. Verfahren nach Anspruch 1, dadurch gekennzeichnet, dass die Umsetzung bei einer Temperatur von 60 bis 300, insbesondere 130 bis 220°C durchgeführt wird.

7. Verfahren nach Anspruch 1, dadurch gekennzeichnet, dass die Verbindungen der Formeln (2), (3) und (4) im Molverhältnis von 2:1:1 bis 2:3:3, insbesondere bis 2:2,5:1,5 umgesetzt werden.

8. Verfahren zur Herstellung von 2,2'-Methylen-bis-[4-(1,1,3,3-tetramethylbutyl)-6-benztriazolylphenol] nach Anspruch 1 durch Umsetzung der Verbindung der Formel (2), worin R₁ Wasserstoff und R₂ 4-(1,1,3,3-tetramethylbutyl) ist, mit einer Verbindung der Formel (3), worin R₃ und R₄ Methyl oder Butyl sind, und Formaldehyd im Molverhältnis von 2:2:1 bis 2:2,2:1,1 bei einer Temperatur von 130 bis 220°C.

## Claims

1. A process for the preparation of a symmetrical 2,2'-methylenebisbenzotriazolylphenol of formula
(1) 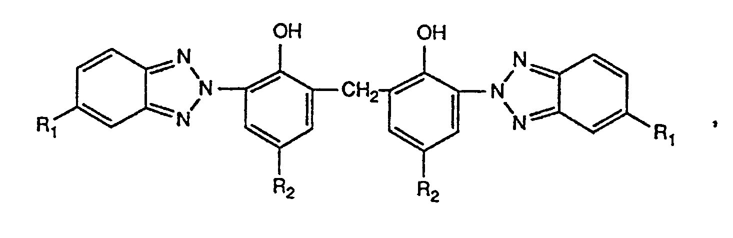 wherein
R₁ is hydrogen, alkyl or alkoxy of 1 to 12 carbon atoms, phenyl, phenylalkyl containing 1 to 4 carbon atoms in the alkyl moiety or halogen, and R₂ is alkyl of 1 to 12 carbon atoms, alkyl of 1 to 12 carbon atoms which is substituted by CO₂H groups, phenyl, phenylalkyl containing 1 to 4 carbon atoms in the alkyl moiety or cycloalkyl of 5 to 8 carbon atoms, in which process a compound of formula
(2) 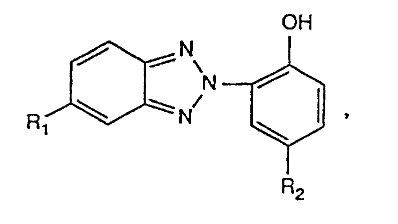 wherein R₁ and R₂ have the given meanings, is reacted with a compound of formula
(3) 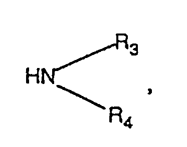 wherein R₃ and R₄ are each independently of the other alkyl of 1 to 4 carbon atoms, and with the compound of formula
(4) CH₂O (formaldehyde), or a polymeric form thereof, to give the corresponding Mannich base, and said Mannich base is treated with a base, which process comprises both reactions being carried out as a one-pot process without a solvent.

2. A process according to claim 1, wherein R₁ is hydrogen, alkyl of 1 to 4 carbon atoms or halogen, and R₂ is alkyl of 1 to 12 carbon atoms.

3. A process according to claim 2, wherein R₁ is hydrogen and R₂ is alkyl of 6 to 12 carbon atoms.

4. A process according to claim 3, wherein R₂ is tert-octyl.

5. A process according to claim 1, wherein R₃ and R₄ are methyl or butyl.

6. A process according to claim 1, wherein the reaction is carried out at a temperature of from 60 to 300°C, in particular from 130 to 220°C.

7. A process according to claim 1, wherein the compounds of formulae (2), (3) and (4) are reacted in a molar ratio of from 2:1:1 to 2:3:3, preferably to 2:2.5:1.5.

8. A process for the preparation of 2,2'-methylenebis[4-(1,1,3,3-tetramethylbutyl)-6-benzotriazolylphenol] according to claim 1 by reacting the compound of formula (2), wherein R₁ is hydrogen and R₂ is 4-(1,1,3,3-tetramethylbutyl), with a compound of formula (3), wherein R₃ and R₄ are methyl or butyl, and formaldehyde, in a molar ratio of from 2:2:1 to 2:2.2:1.1, at a temperature of from 130 to 220°C.

## Revendications

1. Procédé de préparation de 2,2'-méthylène-bis-benzotriazolylphénols symétriques de formule
(1) 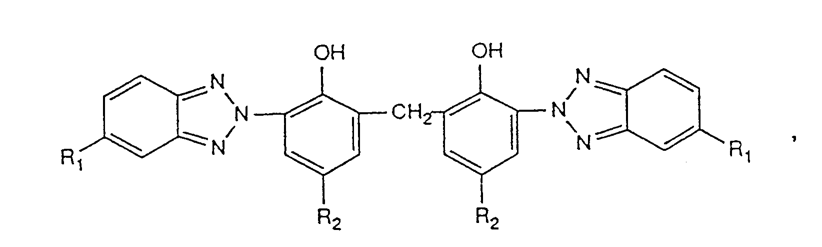 dans laquelle
R₁ est un hydrogène, un reste alkyle ou alcoxy de 1 à 12 atomes de carbone, un reste phényle, un reste phénylalkyle ayant 1 à 4 atomes de carbone dans la partie alkyle, ou un halogène et
R₂ est un reste alkyle de 1 à 12 atomes de carbone, un reste alkyle de 1 à 12 atomes de carbone substitué par des groupes CO₂H, un reste phényle, un reste phénylalkyle ayant 1 à 4 atomes de carbone dans la partie alkyle, ou un reste cycloalkyle de 5 à 8 atomes de carbone, selon lequel on fait réagir un composé de formule
(2) 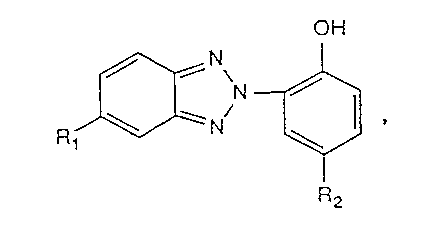 dans laquelle R₁ et R₂ ont les significations indiquées, avec un composé de formule
(3) 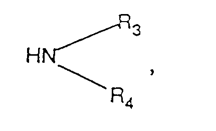 dans laquelle R₃ et R₄ représentent indépendamment l'un de l'autre un reste alkyle de 1 à 4 atomes de carbone, et le composé de formule
(4) CH₂O (formaldéhyde) ou une de ses formes polymères, pour former la base de Mannich correspondante, et on traite cette dernière avec une base, caractérisé en ce que ce que l'on effectue les deux réactions sous forme d'un procédé en un seul récipient en l'absence de solvant.

2. Procédé selon la revendication 1, caractérisé en ce que R₁ est un hydrogène, un alkyle de 1 à 4 atomes de carbone ou un halogène, et R₂ est un alkyle de 1 à 12 atomes de carbone.

3. Procédé selon la revendication 2, caractérisé en ce que R₁ est un hydrogène et R₂ un alkyle de 6 à 12 atomes de carbone.

4. Procédé selon la revendication 3, caractérisé en ce que R₂ est un t-octyle.

5. Procédé selon la revendication 1, caractérisé en ce que R₃ et R₄ sont des restes méthyle ou butyle.

6. Procédé selon la revendication 1, caractérisé en ce que la réaction s'effectue à une température de 60 à 300, en particulier de 130 à 220°C.

7. Procédé selon la revendication 1, caractérisé en ce que l'on fait réagir les composés ayant les formules (2), (3) et (4) dans un rapport molaire de 2:1:1 à 2:3:3, en particulier à 2:2,5:1,5.

8. Procédé de préparation de 2,2'-méthylène-bis[4-(1,1,3,3-tétraméthylbutyl)-6-benzotriazolylphénol] selon la revendication 1 par réaction du composé de formule (2) dans lequel R₁ est l'hydrogène et R₂ est le reste 4-(1,1,3,3-tétraméthylbutyle) avec un composé de formule (3) dans lequel R₃ et R₄ représentent des restes méthyle ou butyle, et le formaldéhyde, en un rapport molaire de 2:2:1 à 2:2,2:1,1, à une température de 130 à 220°C.
